# EUROPEAN PATENT APPLICATION

(11) **EP 1 674 123 A1**
(43) Date of publication of application: **28.06.2006**
(21) Application number: 05253201.7
(22) Date of filing: 25.05.2005
(51) Int. Cl.: A61M 16/00, A61M 16/08, A61M 16/04

(54) **Flow restriction device for a ventilator**

(30) Priority: 24.12.2004 GB 0428298
(71) Applicant: SLE Limited, South Croydon, Surrey CR2 6PL (GB)
(72) Inventor: Mangialaio, Daniel, Norbury, London SW16 4RG (GB)
(74) Representative: Feakins, Graham Allan

(57) **Abstract**

A restrictor device (30) for a patient ventilator (1) is used at the downstream end of an air supply tube (2) and is fitted snugly within an endotracheal tube connector (4). To maintain that pressure in the tube (2), the device (30) has a restricted opening (30A) in it, with loss of back pressure activating an alarm, indicating that the tube (2) has fallen away. To ensure that the alarm is also activated if the restrictor device (30) comes free of the endotracheal tube connector (4), the restricted opening (30A) is located in the downstream end of the device and just above that, openings (30B) are provided which are normally closed off by the inner wall of the connector (4). If the device (30) comes away from the connector (4), then the openings (30B) are exposed, thereby to release the back pressure in the tube (2).

## Description

This invention relates to a restrictor device for a patient ventilator.

With patient ventilators, known practice is to provide a ventilating machine with a tube leading from the machine to a patient via an endotracheal tube (ET tube connector). The purpose of the ET tube connector is to restrict the constant flow of gas coming from the ventilator. This causes a continuous elevated or back pressure in the fluid flow circuit that goes to the patient, known as the "inhalation side" of the circuit, as opposed to the exhalation side. This pressure is continuously monitored and is used to check that there is no leak in the supply to the patient. For example, if the circuit disconnected from the ventilator, then the back pressure would fall and an alarm is activated.

One problem is that if the tubing with the restrictor device fitted itself becomes disconnected from the ET connector, then the back pressure remains and therefore no leak alarm sounds, even though the patient is not receiving ventilation.

Referring first to Figures 1 to 3 of the accompanying drawings,
Fig. 1 is an illustration of the basic patient ventilation circuit,
Fig. 2 is a diagrammatic view of the known restrictor device for use in the ventilator circuit, and
Fig. 3 is a view similar to Fig. 2 but shows the known restrictor device when it has become disconnected.

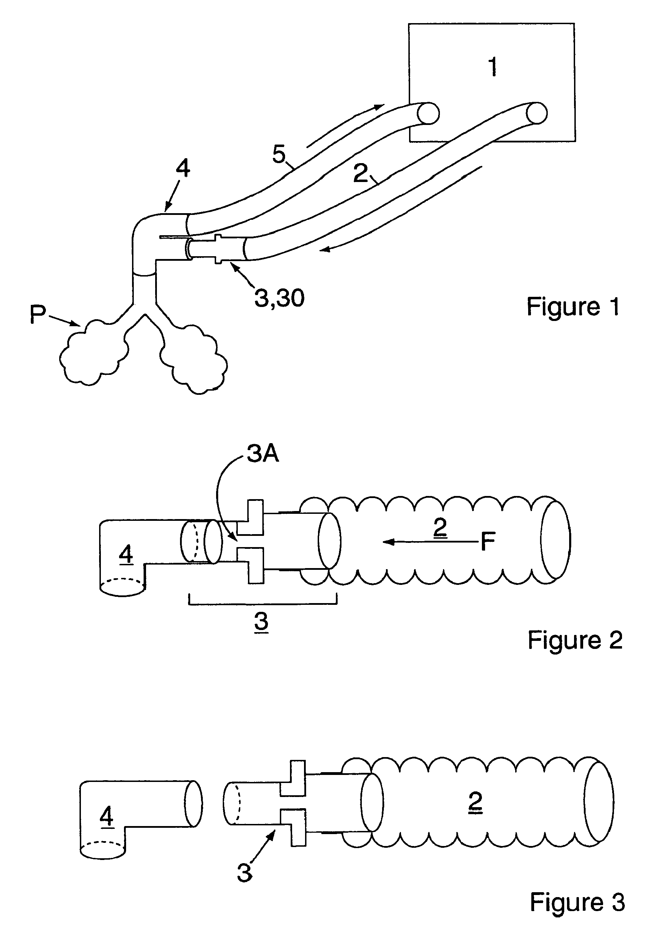

Fig. 1 shows a ventilator apparatus 1 with a tube 2 leading to a known type of restrictor device 3 connected to an ET tube connector 4. Another tube 5 returns the exhaled air from the patient to the ventilator machine 1.

As shown in Fig. 2, the restrictor device 3 is normally connected between the downstream end of the tube 2, the direction of flow being indicated by the arrow F, and is fitted in the upstream open end of the connector.

The restrictor device 3 is provided, intermediate its ends, with an air flow restrictor 3A to provide the required back pressure in the tube 2.

However, as will be realisedfrom Fig. 3, should the restrictor device 3 part company with the connector 4, the disconnected restrictor device will still maintain the back pressure in the tube 2 and therefore no alarm is activated to indicate a leak, even though the patient is not receiving ventilation. This is therefore not a fail-safe situation.

According to the present invention, there is provided a restrictor device for a patient ventilator, wherein the restrictor device is adapted for placement, in use, in a gas flow path between the ventilator and a patient, the restrictor comprising a tube part having a restricted opening at one end of the tube part to provide gas flow through the path downstream of the tube part whilst providing a back pressure upstream of the restrictor device, and there being at least one aperture in one or more side walls of the restrictor device for providing a back pressure decrease upon disconnection of the restrictor, thereby to activate a fluid pressure alarm, the or each aperture being arranged to be closed by an inner wall of a further part to which the tube part is connected in use of the restrictor device, to maintain said back pressure when the device is connected.

The further part to which the tube part is connected may be an endotracheal tube connector.

Thus, the restricted opening of the present restrictor device is located at one end instead of intermediate its ends, as with the known device. Accordingly, in normal circumstances, the restricted opening maintains the back pressure in the tube 2 but the additional aperture(s) in the side wall of the restrictor device which is intended to fit within the ET tube connector is closed off by the inner wall of the ET tube connector when the present restrictor device is fitted in it. However, as soon as the restrictor device parts company with the ET tube connector, it will be immediately seen that the or each aperture vents the tube 2 to such a degree that the back pressure is lost in the tube 2, thereby providing a fail-safe solution to activating the alarm.

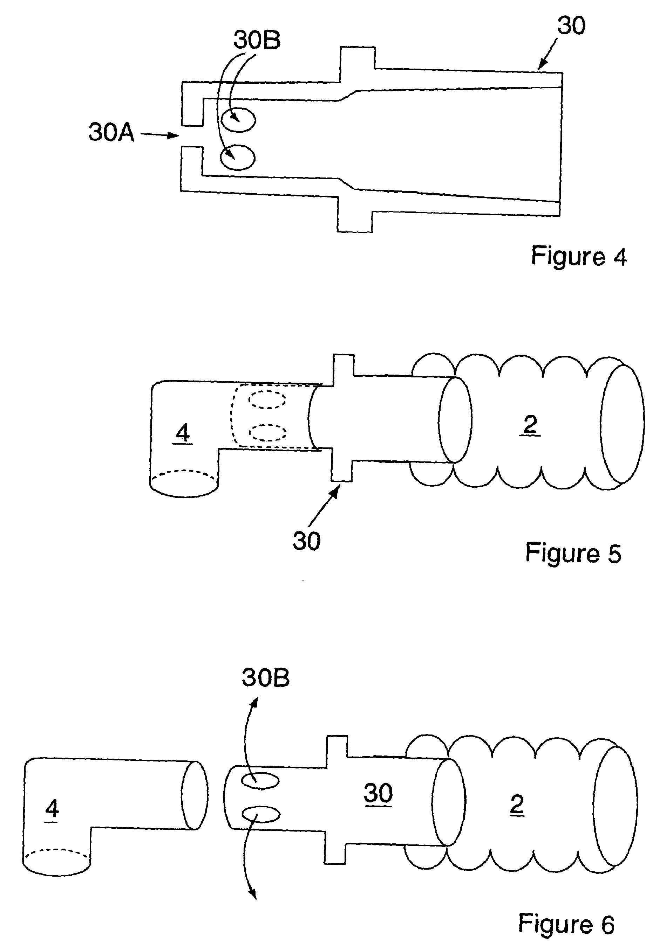

For a better understanding of the invention and to show how the same may be carried into effect, reference will now be made, by way of example, to Figs. 4 to 6 of the accompanying drawings, in which:-
Fig. 4 is a diagrammatic view of the present restrictor device,
Fig. 5 is a diagrammatic view of the present restrictor device fitted in use between the downstream end of the supply tube and the upstream of the ET tube connector, and
Fig. 6 illustrates the situation when the present restrictor device parts company with the ET tube connector.

Referring to Figs. 4 to 6, the present restrictor device is indicated by the reference numeral 30 and it will be seen that it has a similar external configuration to the known type of connector device 3. The restrictor device 30 is accordingly fitted in similar fashion in the downstream end of the tube 2 leading from the ventilator 1, whilst the downstream end of the restrictor device 30 is fitted in the upstream end of the ET tube connector 4.

As explained above, the main departure from the known device is that the restricted opening 30A is relocated at the downstream end of the restrictor device 30, whilst apertures 30B are provided in that tubular part of the restrictor device 30 which fits snugly, as a friction fit, in use inside the open end of the ET tube connector 4, as shown in Fig. 5. In other words, the apertures 30B are closed or blocked by the inside wall of the ET tube connector 4. In such a case, of course, the back pressure in the tube 2 is maintained.

However, as shown in Fig. 6, as soon as the restrictor device 3 parts company with the ET tube connector 4, the apertures 30B are opened, thereby allowing the back pressure to fall to activate the alarm.

## Claims

1. A restrictor device (30) for a patient ventilator (1), wherein the restrictor device is adapted for placement, in use, in a gas flow path (2) between the ventilator and a patient, the restrictor device comprising a tube part having a restricted opening (30A); **characterised in that** the restricted opening (30A) is at one end of the tube part to provide gas flow through the path downstream of the tube part whilst providing a back pressure upstream of the restrictor device (30), and there being at least one aperture (30B) in one or more side walls of the restrictor device for providing a back pressure decrease upon disconnection of the restrictor, thereby to activate a fluid pressure alarm, the or each aperture being arranged to be closed by an inner wall of a further part to which the tube part is connected in use of the restrictor device, to maintain said back pressure when the device is connected.

2. A device according to claim 1 in combination with an endotracheal tube connector (4), to which the device is fitted.

3. The combination according to claim 2, wherein the restrictor device (30) is so fitted in the endotracheal tube connector (4) that the or each aperture (30B) of the restrictor device is closed by an inner wall of the endotracheal tube device (4), the restricted opening (30A) in the restrictor device lying within the endotracheal tube device.
